(19) Europäisches Patentamt
European Patent Office
Office européen des brevets

(11)  **EP 3 578 100 A1**

(12)  # EUROPEAN PATENT APPLICATION

(43) Date of publication:
**11.12.2019  Bulletin 2019/50**

(51) Int Cl.:
**A61B 5/021** (2006.01)       **A61B 5/00** (2006.01)

(21) Application number: **18175931.7**

(22) Date of filing: **05.06.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
• **MUEHLSTEFF, Jens**
  **5656 AE Eindhoven (NL)**

• **BRESCH, Erik**
  **5656 AE Eindhoven (NL)**
• **VAN DALEN, Jan**
  **5656 AE Eindhoven (NL)**
• **JUNYENT MORA, Ignasi**
  **5656 AE Eindhoven (NL)**
• **SCHMITT, Lars**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54)  **METHOD AND APPARATUS FOR ESTIMATING A TREND IN A BLOOD PRESSURE SURROGATE**

(57)  According to an aspect, there is provided a computer-implemented method of estimating a trend in a blood pressure surrogate, the method comprising obtaining a set of blood pressure surrogate measurement values of a blood pressure surrogate for a subject; obtaining, for each blood pressure surrogate measurement value, an error value indicating a measurement error for the blood pressure surrogate measurement value; and analysing the set of blood pressure surrogate measurement values and the respective error values using Bayesian inference to determine a trend (4) in the blood pressure surrogate over time.

(a)

(b)

Fig. 1

**EP 3 578 100 A1**

**Description**

FIELD OF THE INVENTION

This disclosure relates to the analysis of a measure that can be used as a surrogate for blood pressure of a subject, and in particular relates to a method and apparatus for estimating a trend in the blood pressure surrogate.

BACKGROUND OF THE INVENTION

So-called quantified self makes use of technology to measure and gather data or information on the activities of a person's daily life, such as movements, food consumed, amount of exercise, mood, etc. The insight that characteristics of the skin of the person can provide in the monitoring of vital signs (such as heart rate) has led to the introduction of wearables, like smartwatches and wristbands, into the market.

Alongside these wearables, so-called Smart Mirrors are being considered in which a standard mirror (e.g. for use in a bathroom environment) is enhanced with one or more sensors to enable the measurement of a number of vital signs by monitoring skin from a certain distance (i.e. contactless measurement) or by making use of touch (i.e. contact-based measurement, such as touch).

The wearables and smart mirrors that are intended to provide health-related data typically only measure (or enable the measurement of) the heart rate, the heart rate variability, the breathing rate and the oxygen saturation (SpO2) levels of the person.

Blood pressure is another useful physiological characteristic of a person that could be measured, and it is desirable to use wearables or smart mirrors to measure blood pressure in an unobtrusive way. It is typically difficult to obtain a blood pressure measurement remotely (i.e. contactlessly), so instead one or more other physiological characteristics are measured that can provide an indication of the blood pressure of the subject, or an indication of the trend in the blood pressure over time. One such 'surrogate' physiological characteristic is the pulse wave velocity (PWV). Pulse wave velocity (PWV) is the speed at which a pressure pulse propagates along the arterial tree of a person, and can be measured by observing the time taken for a pulse to move between two measurement points (known as the pulse transit time, PTT) that are a known distance apart. The PWV is given by the distance between the measurement points divided by the PTT. By itself, PWV is an indicator of arterial pathologies and cardiovascular health.

PWV is considered to be a useful indicator of blood pressure in a person, and PWV is therefore viewed as a good characteristic to use in a low-hassle unobtrusive monitor of blood pressure. In particular, it is considered that there is a correlation between the trend of PWV for a person and the trend of blood pressure for the person.

There are several techniques used to measure PWV in a non-invasive way. A common technique makes use of photoplethysmography (PPG) at two sites of the body to detect blood volume changes as pulse signals, with PWV being calculated from the distance between the sensors divided by the time difference between the detected pulses. Another option uses electrocardiography (ECG) in combination with another sensor (e.g. PPG, ultrasound, impedance cardiography, etc.), in which the pulse arrival time (PAT) is measured (i.e. the time at which a pulse arrives at a particular point), and the PAT is calculated by measuring the time difference between the R-peak of the ECG signal and characteristic points of the signal obtained with the other sensor.

SUMMARY OF THE INVENTION

As noted above, PAT, PTT and/or PWV measurements (or other physiological characteristic measurements) obtained over time can be used as an indicator of a trend in blood pressure (which can be context specific). One approach is to infer a trend in the blood pressure by applying a classical Least-Means-Square (LMS) method to the PAT, PTT and/or PWV measurements. However, any measurement of a physiological characteristic is accompanied by measurement errors, which have an impact on the reliability of the results. This is particularly the case when using the LMS method to infer a blood pressure trend from a trend in PAT, PTT and/or PWV as the reliability of the measurements is typically not taken into account.

Measurement errors or measurement uncertainty (i.e. a range in which the actual measurement value may lie) is particularly a problem for contactless measurements and for measurements obtained in a short time window (e.g. when the person is standing in front of a mirror).

Therefore there is a desire for improvements in the estimation of a trend from measurements of a blood pressure surrogate (such as PAT, PTT and/or PWV) particularly where the measurements of the blood pressure surrogate may have measurement errors.

According to a first specific aspect, there is provided a computer-implemented method of estimating a trend in a blood pressure surrogate, the method comprising obtaining a set of blood pressure surrogate measurement values of a blood pressure surrogate for a subject; obtaining, for each blood pressure surrogate measurement value, an error value indicating a measurement error for the blood pressure surrogate measurement value; and analysing the set of blood pressure surrogate measurement values and the respective error values using Bayesian inference to determine a trend in the blood pressure surrogate over time. This method improves the estimation of a trend from measurements of a blood pressure surrogate where the measurements of the blood pressure surrogate may have measurement errors.

In some embodiments, the step of analysing comprises correcting each of the blood pressure surrogate measurement values in the set according to a point in a circadian rhythm of the subject at which each blood pressure surrogate measurement value was measured; and using Bayesian inference to determine the trend in the blood pressure surrogate over time by analysing the corrected blood pressure surrogate measurement values and the respective error values.

In these embodiments, the step of correcting can comprise obtaining measurements of a circadian rhythm surrogate of the subject; analysing the measurements of the circadian rhythm surrogate to identify a circadian rhythm of the subject; based on the identified circadian rhythm, determining respective points in the circadian rhythm at which each of the blood pressure surrogate measurement values in the set was measured; determining a respective correction for each of the blood pressure surrogate measurement values in the set based on the determined respective points in the circadian rhythm; and applying the determined respective corrections to the blood pressure surrogate measurement values in the set.

In these embodiments, the step of determining a respective correction can be based on (i) a linear function of the circadian rhythm surrogate and the blood pressure surrogate; (ii) a mapping function relating the circadian rhythm surrogate to the blood pressure surrogate; (iii) a predetermined set of corrections for respective points in the circadian rhythm; or (iv) data for a population of other subjects.

In these embodiments the circadian rhythm surrogate can be heart rate, body temperature and/or physical activity level.

In some embodiments, the step of obtaining the set of blood pressure surrogate measurement values comprises, for each blood pressure surrogate measurement value: obtaining a plurality of blood pressure surrogate measurement values during a time window; evaluating the blood pressure surrogate measurement values obtained during the time window to determine if the blood pressure surrogate is in a steady state; and including a blood pressure surrogate measurement value obtained when the blood pressure surrogate is in a steady state in the set of blood pressure surrogate measurement values.

In alternative embodiments, the step of obtaining the set of blood pressure surrogate measurement values comprises, for each blood pressure surrogate measurement value: obtaining a plurality of blood pressure surrogate measurement values during a time window; evaluating the blood pressure surrogate measurement values obtained during the time window to determine if the blood pressure surrogate is in a steady state; and if the blood pressure surrogate is not in a steady state, extrapolating the blood pressure surrogate measurement values obtained during the time window to estimate the blood pressure surrogate measurement value in the steady state.

In some embodiments, the method further comprises the step of providing instructions to the subject to perform a procedure or exercise prior to, or during, a measurement of the blood pressure surrogate.

In these embodiments, the method can further comprise the step of: monitoring the subject to determine if the subject is correctly performing the procedure or exercise; and providing feedback or further instructions to the subject to assist the subject in correctly performing the procedure or exercise.

In some embodiments, the blood pressure surrogate is pulse arrival time, PAT, and the step of analysing comprises: obtaining measurements of a heart rate of the subject; estimating a pre-ejection period, PEP, for each PAT measurement value from the heart rate at the time of the PAT measurement; subtracting the estimated PEP from the respective PAT measurement value to determine a set of corrected PAT measurement values; and using Bayesian inference to determine the trend in the PAT over time by analysing the corrected PAT measurement values and the respective error values.

In alternative embodiments, the blood pressure surrogate is any one of pulse wave velocity, PWV, pulse arrival time, PAT, and pulse transit time, PTT, heart rate, blood volume distribution, pulse morphology changes, and gross body movements.

In some embodiments, the step of obtaining the set of blood pressure surrogate measurement values comprises obtaining the blood pressure surrogate measurement values using one or more of a photoplethysmography, PPG, sensor, an electrocardiography, ECG, sensor, an accelerometer, an image capture device, a video capture device, a radar-based sensor, a laser-based Doppler sensor, a sensor that measures interferences and a Moiré sensor.

In some embodiments, the method is performed by a smart mirror that comprises one or more sensors for obtaining the set of blood pressure surrogate measurement values.

In some embodiments, the method further comprises the step of using the determined trend in the blood pressure surrogate over time as an indicator of a trend in blood pressure.

According to a second aspect, there is provided a computer program product comprising a computer readable medium having computer readable code embodied therein, the computer readable code being configured such that, on execution by a suitable computer or processor, the computer or processor is caused to perform the method according to the first aspect or any embodiment thereof.

According to a third specific aspect, there is provided an apparatus for estimating a trend in a blood pressure surrogate, the apparatus comprising a processing unit configured to obtain a set of blood pressure surrogate measurement values of a blood pressure surrogate for a subject; obtain, for each blood pressure surrogate measurement value, an error value indicating a measurement error for the blood pressure surrogate measurement value; and analyse the set of blood pressure surrogate measurement values and the respective error values using Bayesian inference to determine a trend in the blood pressure surrogate over time. This apparatus provides an improved estimation of a trend from measurements of a blood pressure surrogate where the measurements of the blood pressure surrogate may have measurement errors.

In some embodiments, the processing unit is configured to analyse the set of blood pressure surrogate measurement values and the respective error values using Bayesian inference to determine a trend in the blood pressure surrogate over time by correcting each of the blood pressure surrogate measurement values in the set according to a point in a circadian rhythm of the subject at which each blood pressure surrogate measurement value was measured; and using Bayesian inference to determine the trend in the blood pressure surrogate over time by analysing the corrected blood pressure surrogate measurement values and the respective error values.

In these embodiments, the processing unit is configured to correct each of the blood pressure surrogate measurement values in the set by obtaining measurements of a circadian rhythm surrogate of the subject; analysing the measurements of the circadian rhythm surrogate to identify a circadian rhythm of the subject; based on the identified circadian rhythm, determining respective points in the circadian rhythm at which each of the blood pressure surrogate measurement values in the set was measured; determining a respective correction for each of the blood pressure surrogate measurement values in the set based on the determined respective points in the circadian rhythm; and applying the determined respective corrections to the blood pressure surrogate measurement values in the set.

In these embodiments, the processing unit can be configured to determine a respective correction based on (i) a linear function of the circadian rhythm surrogate and the blood pressure surrogate; (ii) a mapping function relating the circadian rhythm surrogate to the blood pressure surrogate; (iii) a predetermined set of corrections for respective points in the circadian rhythm; or (iv) data for a population of other subjects.

In these embodiments the circadian rhythm surrogate can be heart rate, body temperature and/or physical activity level.

In some embodiments, the processing unit is configured to obtain the set of blood pressure surrogate measurement values by, for each blood pressure surrogate measurement value: obtaining a plurality of blood pressure surrogate measurement values during a time window; evaluating the blood pressure surrogate measurement values obtained during the time window to determine if the blood pressure surrogate is in a steady state; and including a blood pressure surrogate measurement value obtained when the blood pressure surrogate is in a steady state in the set of blood pressure surrogate measurement values.

In alternative embodiments, the processing unit is configured to obtain the set of blood pressure surrogate measurement values comprises, for each blood pressure surrogate measurement value: obtaining a plurality of blood pressure surrogate measurement values during a time window; evaluating the blood pressure surrogate measurement values obtained during the time window to determine if the blood pressure surrogate is in a steady state; and if the blood pressure surrogate is not in a steady state, extrapolating the blood pressure surrogate measurement values obtained during the time window to estimate the blood pressure surrogate measurement value in the steady state.

In some embodiments, the processing unit is further configured to provide instructions to the subject, via a user interface, to perform a procedure or exercise prior to, or during, a measurement of the blood pressure surrogate.

In these embodiments, the processing unit can be further configured to monitor the subject to determine if the subject is correctly performing the procedure or exercise; and provide feedback or further instructions to the subject, via the user interface, to assist the subject in correctly performing the procedure or exercise.

In some embodiments, the blood pressure surrogate is pulse arrival time, PAT, and the processing unit is configured to analyse the set of blood pressure surrogate measurement values and the respective error values using Bayesian inference to determine a trend in the blood pressure surrogate over time by: obtaining measurements of a heart rate of the subject; estimating a pre-ejection period, PEP, for each PAT measurement value from the heart rate at the time of the PAT measurement; subtracting the estimated PEP from the respective PAT measurement value to determine a set of corrected PAT measurement values; and using Bayesian inference to determine the trend in the PAT over time by analysing the corrected PAT measurement values and the respective error values.

In alternative embodiments, the blood pressure surrogate is any one of pulse wave velocity, PWV, pulse arrival time, PAT, and pulse transit time, PTT, heart rate, blood volume distribution, pulse morphology changes, and gross body movements.

In some embodiments, the processing unit is configured to obtain the set of blood pressure surrogate measurement values by obtaining the blood pressure surrogate measurement values using one or more of a photoplethysmography, PPG, sensor, an electrocardiography, ECG, sensor, an accelerometer, an image capture device, a video capture device, a radar-based sensor, a laser-based Doppler sensor, a sensor that measures interferences and a Moiré sensor.

In some embodiments, the apparatus is a smart mirror that comprises one or more sensors for obtaining the set of blood pressure surrogate measurement values.

In some embodiments, the processing unit is further configured to use the determined trend in the blood pressure surrogate over time as an indicator of a trend in blood pressure.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 includes two plots illustrating different approaches for determining a trend from a series of measurements;
Fig. 2 is a block diagram of an apparatus according to various embodiments;
Fig. 3 is an illustration of an apparatus in the form of a smart mirror according to various embodiments;
Fig. 4 is a flow chart illustrating a method according to various embodiments;
Fig. 5 is a plot illustrating the influence of the circadian rhythm on blood pressure;
Fig. 6 is a plot illustrating changes in PAT over time in response to a physical activity;
Fig. 7 is a pair of plots illustrating an effect of breathing on PAT;
Fig. 8 is a plot illustrating PAT against heart rate for a series of measurements;
Fig. 9 is a plot illustrating the difference between PAT and pre-ejection period (PEP) against heart rate for a series of measurements;
Fig. 10 is a plot illustrating heart rate over time for a series of measurements;
Fig. 11 is a plot illustrating systolic blood pressure over time for a series of measurements;
Fig. 12 is a plot illustrating PAT over time for a series of measurements; and
Fig. 13 is a plot illustrating the difference between PAT and PEP over time for a series of measurements.

DETAILED DESCRIPTION OF EMBODIMENTS

A "blood pressure surrogate", as used herein, is any physiological characteristic or characteristic of the body whose measurements can provide an indication of the blood pressure of a subject, or an indication of changes in the blood pressure of the subject over time (i.e. a trend). As noted above, one blood pressure surrogate is pulse wave velocity, PWV. Other blood pressure surrogates that can be used according to the embodiments described herein include, for example, pulse arrival time, PAT, pulse transit time, PTT, heart rate, blood volume distribution (e.g. in the face), pulse morphology changes, gross body movements (e.g. measured using a ballistocardiogram). Thus, PAT, PTT and/or PWV (or other physiological characteristic) measurements obtained over time can be used to as an indicator of a trend in blood pressure, although the measurement errors associated with the measurement of these blood pressure surrogates can have a significant impact on the reliability of the resulting trend. Measurement errors or measurement uncertainty (i.e. a range in which the actual measurement value may lie) is particularly a problem for contactless measurements and for measurements obtained in a short time window (e.g. when the person is standing in front of a mirror), for example measurements obtained when a subject is briefly in front of a smart mirror.

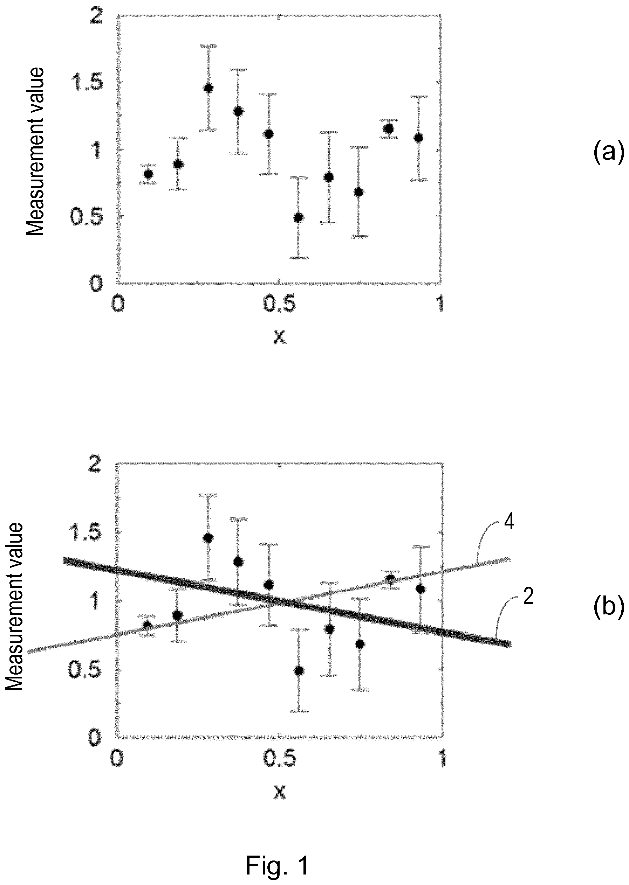

One approach is to infer a trend in a set of measurements is to use a Least-Means-Square (LMS) method. However, a LMS method does not take the reliability (or error) of the measurements into account, and this can reduce the accuracy of the trend line. In contrast, a Bayesian inference method does take the measurement error into account and can therefore provide a more reliable indication of a trend. The Bayesian approach enables an objectified method to quantitatively discriminate a linear versus a constant trend. The differences between a LMS approach and a Bayesian approach are illustrated in Fig. 1.

Fig. 1(a) shows an exemplary set of ten measurements, with each measurement having a respective error bar showing the measurement error (uncertainty) for each measurement. It can be seen that the error bars vary across the different measurements. The underlying trend in the measurements in Fig. 1(a) is positive (i.e. the measurements are generally increasing). Fig. 1(b) shows two different trend lines derived from the set of measurements in Fig. 1(a). The first line, labelled 2, is derived using a standard LMS procedure and suggests that there is a negative trend. The second line, labelled 4, is derived using Bayesian inference and takes the measurement error (the error bars) into account. In particular, the Bayesian approach makes use of the much lower measurement error for the first and ninth measurements (as counted from the left hand side of the plot) to (correctly) identify the positive measurement trend. In addition, to discriminate the underlying trend in the set of measurements, the Bayesian approach can provide a quantified measure as a probability of an e.g. linear trend vs. constant values (the "odd ratio"). The value of this odd ratio can be used as a threshold. This can be expressed via the obtained measurements $\{x, y\}$ and basic statistical considerations with $D = D(y)$ such as the odd ratio $O$ is given by:

$$O = \frac{1}{2}\left(\frac{2\pi\rho^2}{N\overline{\Delta x^2}}\right)^{1/2} \frac{1}{\left(1 + \overline{\Delta D \Delta x}/\overline{\Delta x^2}\right)^{3/2}} \cdot exp\left\{\frac{N}{2\rho^2} \cdot \frac{\overline{\Delta x \Delta D}^2}{\overline{\Delta x^2}}\right\} \tag{1}$$

where $N$ is the number of observations, $\rho$ is defined via the term $\sum_i^N 1/s_i^2 = N/\rho^2$ with $s_i$ the approximation of the standard deviation for the observation $y_i$, $\Delta x$ is given by $x_i$ minus the average of all $x_i$ and $\Delta D$ is defined as $y_i$ minus the average of all $y_i$. Values of $\ln(O) > 5$ are typically considered as "overwhelming" evidence of the existence of the linear trend, 2.5 to 5 as a strong indication of the existence of the linear trend, 1 to 2.5 as a positive indication of the existence of the linear trend and $< 1$ as a linear trend is not indicated or present in the measurements. This measure can be also used as feedback to the subject on how strong (i.e. how likely) a trend actually is.

Thus, it is proposed to use a Bayesian inference approach to infer a blood pressure from measurements of a blood pressure surrogate, such as pulse wave velocity (PWV), pulse arrival time (PAT) and/or pulse transit time (PTT).

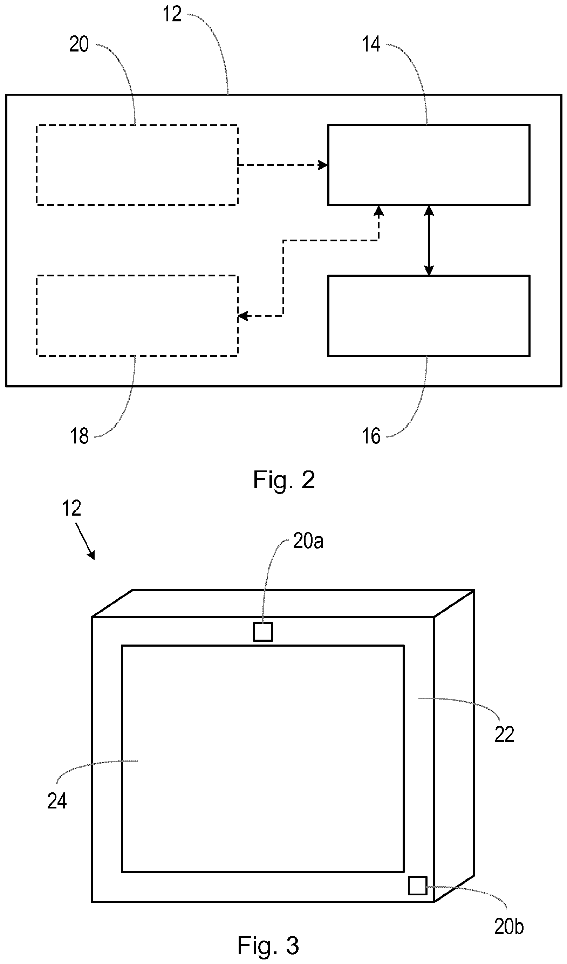

An apparatus that can be used to determine a blood pressure surrogate trend from measurements of one or more blood pressure surrogates is shown in Fig. 2. The apparatus 12 includes a processing unit 14 that controls the operation of the apparatus 12 and that can be configured to execute or perform the methods described herein. The processing unit 14 can be implemented in numerous ways, with software and/or hardware, to perform the various functions described herein. The processing unit 14 may comprise one or more microprocessors or digital signal processor (DSPs) that may be programmed using software or computer program code to perform the required functions and/or to control components of the processing unit 14 to effect the required functions. The processing unit 14 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 14 is connected to a memory unit 16 that can store data, information and/or signals for use by the processing unit 14 in controlling the operation of the apparatus 12 and/or in executing or performing the methods described herein. In some implementations the memory unit 16 stores computer-readable code that can be executed by the processing unit 14 so that the processing unit 14 performs one or more functions, including the methods described herein. The memory unit 16 can comprise any type of non-transitory machine-readable medium, such as cache or system memory including volatile and nonvolatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

The apparatus 12 optionally includes interface circuitry 18 for enabling a data connection to and/or data exchange with other devices, including any one or more of servers, databases, user devices, and sensors. The connection may be direct or indirect (e.g. via the Internet), and thus the interface circuitry 18 can enable a connection between the apparatus 12 and a network, such as the Internet, via any desirable wired or wireless communication protocol. For example, the interface circuitry 18 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). The interface circuitry 18 can be connected to the processing unit 14.

In some embodiments, the apparatus 12 may also include a user interface (not shown in Fig. 2) that includes one or more components that enables a user of apparatus 12 (e.g. the subject whose blood pressure trend is being measured or estimated) to input information, data and/or commands into the apparatus 12, and/or enables the apparatus 12 to output information or data to the user of the apparatus 12. The user interface can comprise any suitable input component(s), including but not limited to a keyboard, keypad, one or more buttons, switches or dials, a mouse, a track pad, a touchscreen, a stylus, a camera, a microphone, etc., and the user interface can include any suitable output component(s), including but not limited to a display screen, one or more lights or light elements, one or more loudspeakers, a vibrating element, etc.

To determine a blood pressure surrogate trend, measurements of one or more blood pressure surrogates are required. These measurements (or measurement values) are obtained using one or more blood pressure surrogate sensor(s). The blood pressure surrogate sensor(s) may be part of the apparatus 12 (e.g. as shown by blood pressure surrogate sensor 20 in Fig. 2), or the blood pressure surrogate sensor(s) may be separate from the apparatus 12. In the latter case, the apparatus 12 can be connected to the blood pressure surrogate sensor(s) to receive the blood pressure surrogate measurements directly from the blood pressure surrogate sensor(s), or the apparatus 12 can receive the blood pressure surrogate measurements via the interface circuitry 18. The blood pressure surrogate measurement values can be provided to the processing unit 14 for processing or the measurement values can be stored in the memory unit 16 for processing by the processing unit 14 at a later time.

The blood pressure surrogate sensor 20 can be any type of sensor that can measure a blood pressure surrogate of a subject. The blood pressure surrogate sensor 20 can output a measurement signal indicating the blood pressure surrogate, or the blood pressure surrogate sensor 20 can output a measurement signal that can be analysed or processed to determine the blood pressure surrogate. In some embodiments, as noted above, the blood pressure surrogate can be the PWV, PAT and/or PTT of a subject, heart rate, blood volume distribution (e.g. in the face), pulse morphology changes, gross body movements (e.g. measured using a ballistocardiogram), or any other physiological characteristic that can be used as a surrogate measurement for blood pressure (i.e. any other physiological characteristic whose changes correlate or approximately correlate with changes in blood pressure).

To measure, for example, PWV or PTT, the blood pressure surrogate sensor 20 can comprise multiple sensing elements (for example two sensing elements to be placed on different locations on the body of the subject to measure a pulse signal at each location), and these sensing elements may be the same or different types.

In some embodiments, the blood pressure surrogate sensor 20 is or includes one or more photoplethysmogram (PPG) sensors. Each PPG sensor comprises a light source for illuminating a part of the body of the subject (e.g. a finger, wrist, ear, forehead, etc., and a light sensor for measuring the light reflected and/or absorbed by the skin/tissue near the light source. The light sensor outputs a measurement signal representing the measured light intensity, and this measurement signal can be processed to identify blood volume changes as a pulse signal. Typically the PPG sensor is placed in contact with the skin. The PPG sensor measures characteristics of the blood volume change in superficial skin tissue (i.e. near the surface of the skin), and characteristics representing the pulse or heart beat of the subject, such as the arrival time of a pulse, can be extracted from the PPG measurement signal.

In other embodiments, the blood pressure surrogate sensor 20 can be one or more electrocardiogram (ECG) sensors that comprises one or more electrodes that are placed on the body of the subject or that can be close to the body and that measure the electrical activity of the heart. In some embodiments, capacitive ECG electrodes can be used that can measure an ECG signal when the subject is in contact with the electrodes. For example, electrodes can be provided on the floor or on a set of weighing scales, and an ECG signal can be measured while the subject stands on the electrodes on the floor or weighing scales.

In some embodiments, the blood pressure surrogate sensor 20 can include two PPG sensors that are to be placed at different locations on the body of the subject to measure the arrival time of a pulse at each location (where one PPG sensor is placed downstream of the other PPG sensor to enable the arrival times of the same pulse at different locations to be measured). In other embodiments, the blood pressure surrogate sensor 20 can include a PPG sensor and an ECG sensor. The ECG sensor is used to measure the electrical activity of the heart, and in particular the R-peak, and the PPG sensor is used to measure the time of arrival of a pulse at the position of the PPG sensor.

In other embodiments, the blood pressure surrogate sensor 20 can be an accelerometer or other type of movement sensor that is placed in contact with a part of the body of the subject (e.g. chest, neck, wrist, finger, etc.) and that measures the accelerations (or more generally the movements) of the part of the body. An accelerometer (or other type of movement sensor) can be sensitive enough to movements of the skin caused by the pulsing of blood through blood vessels beneath the skin that the arrival of a pulse of blood can be extracted from the measurement signal. In the case of an accelerometer, the accelerometer can measure the accelerations along three orthogonal axes (e.g. labelled X, Y and Z) and output three signals, each representing the accelerations along a respective one of the axes, or output a single signal that is a composite of the accelerations measured along the three orthogonal axes. The measurement signal from the accelerometer can be processed using signal analysis techniques known in the art to extract movements due to a heart beat/pulsing of blood, and thus the blood pressure surrogate measurement values can be derived from the acceleration measurements. Other types of movement sensor from which a heart rate measurement can be obtained include a gyroscope that measures changes in rotation and orientation. Those skilled in the art will be aware of other types of movement sensor that can be used to measure blood pressure surrogates.

It will be appreciated that an accelerometer (or other movement sensor) can be used in combination with a PPG sensor (in place of a second PPG sensor) or with an ECG sensor (in place of a PPG sensor) to measure the blood pressure surrogate.

It will be appreciated that the blood pressure surrogate sensors 20 outlined above require direct or physical contact with the subject. However, in some embodiments, the blood pressure surrogate sensor 20 measures the blood pressure surrogate contactlessly, i.e. without requiring physical contact with the subject. For example, the blood pressure surrogate sensor 20 can be an image sensor (e.g. a camera) for capturing images or a video sequence. The images or video sequence can be processed using image analysis techniques to extract the blood pressure surrogate measurements. For example, the images or video sequence can be processed to identify areas of skin within the images or video sequence, and those areas analysed to extract a PPG signal for the skin. This is known as remote PPG (rPPG), and techniques for analysing images or a video sequence to extract a PPG signal are known in the art. Besides rPPG signals, a camera can also (or alternatively) be used to detect local tissue (skin) movements (e.g. in the region of the carotid artery) due to pulse signals as well as gross body movements caused by the pumping action of the heart. Alternatively, a radar-based sensor can be used to measure the movements of the skin (i.e. due to pulses). Other types of sensors that can be used to measure a blood pressure surrogate include laser-based Doppler sensors, sensors that use coherent light to measure interferences (speckle effects), and Moiré sensors.

The blood pressure surrogate sensor 20 can operate with any suitable sampling frequency to provide measurements of the blood pressure surrogate, for example 10 Hertz (Hz) or 50 Hz. This means that, e.g. in the case of an accelerometer, the accelerometer can output an acceleration measurement every 1/10$^{th}$ of a second or 1/50$^{th}$ of a second.

The apparatus 12 can be any type of electronic device or computing device. For example the apparatus 12 can be, or be part of, a server, a computer, a laptop, a tablet, a smartphone, etc. In some embodiments, the apparatus 12 can be, or be part of, a smart mirror or other household or domestic device that is used by a subject from time to time. For example, the apparatus 12 may be part of a smart mirror that is placed in a bathroom of the house of the subject. Alternatively, the apparatus 12 may be part of a television. As another alternative, the apparatus 12 can be, or be part of, a wearable device that can be worn by the subject. For example, the wearable device may be a watch, smart watch, a bracelet, a necklace, another type of jewellery item, an item of clothing, etc.

It will be appreciated that a practical implementation of an apparatus 12 may include additional components to those shown in Fig. 1. For example the apparatus 12 may also include a power supply, such as a battery, or components for enabling the apparatus 12 to be connected to a mains power supply.

Fig. 3 shows an exemplary apparatus 12 implemented in the form of a smart mirror. Thus, in Fig. 3 the apparatus 12 comprises a frame 22, a mirror surface 24 and a blood pressure surrogate sensor 20. The mirror surface 24 may be a conventional reflective surface. Alternatively, the mirror surface 24 may be in the form of a display screen that displays a video sequence obtained by a video capture device (e.g. a camera). The video capture device will be oriented with respect to the smart mirror so that it captures a video sequence of anything in front of the smart mirror. The captured video sequence is displayed on the mirror surface 24 (i.e. display screen) in real time to provide a 'reflection' of a subject if a subject is in front of the mirror surface 24.

Fig. 3 shows two possible blood pressure surrogate sensors 20 (although in some embodiments only one of these may be present in the apparatus 12). A first blood pressure surrogate sensor 20a is a camera or other image capture device that can be located in the frame 22 (although other locations are possible) that obtains images or a video sequence that can be processed or analysed using remote PPG techniques to extract blood pressure surrogate measurement values. In embodiments where the mirror surface 24 is a display screen, the camera 20a can also be used to provide the video sequence to be displayed on the mirror surface 24. A second blood pressure surrogate sensor 20b is a PPG sensor that is also located in the frame 22 (although again other locations are possible). The light source and light sensor in the PPG sensor 20b can be arranged in the frame 22 so that the subject can place a finger or other body part in contact with them to enable a PPG signal to be obtained.

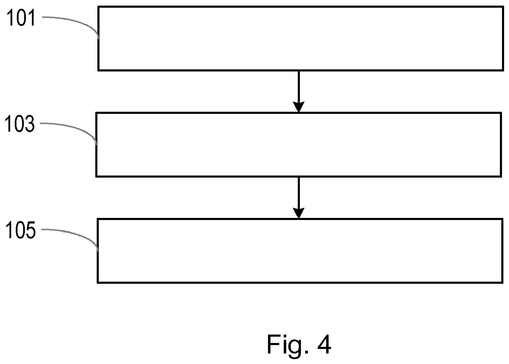

A method of estimating a trend in a blood pressure surrogate for a subject is shown in the flow chart of Fig. 4. The method can be performed by the apparatus 12, and particularly by the processing unit 14, for example in response to executing computer-readable code that is stored in the memory unit 16 or stored on some other suitable computer-readable medium, such as an optical disk, a solid state storage device, a magnetic-disk based storage device, etc.

In step 101 a set of measurement values of a blood pressure surrogate for the subject are obtained. In some embodiments, the blood pressure surrogate is any of PWV, PAT, PTT, or any other physiological characteristic that can be used as a surrogate measurement for blood pressure (i.e. any other physiological characteristic whose changes correlate or approximately correlate with changes in blood pressure).

In some embodiments, a set of measurement values for two or more blood pressure surrogates can be obtained. In this case, the blood pressure surrogates can be two or more of PWV, PAT, PTT and any other physiological characteristic that can be used as a surrogate measurement for blood pressure. The set of measurement values of the blood pressure surrogate include measurement values for the blood pressure surrogate obtained at different times over a period of time (e.g. the set may include measurement values for a number of hours, days, weeks, etc.). In embodiments where the apparatus 12 includes a blood pressure surrogate sensor 20, step 101 can comprise obtaining a set of blood pressure surrogate measurement values using the blood pressure surrogate sensor 20 and storing them (e.g. in memory unit 16) for processing at a later stage. In embodiments where the apparatus 12 does not include a blood pressure surrogate sensor 20, step 101 can comprise receiving a set of measurement values from another device (e.g. that includes a blood pressure surrogate sensor) or retrieving a set of measurement values from the memory unit 16.

In step 103 an error value is obtained for each of the blood pressure surrogate measurement values obtained in step 101. Each error value indicates a measurement error for the corresponding blood pressure surrogate measurement value, i.e. each error value indicates the level of accuracy of the corresponding measurement value. For example, the error value can indicate the measurement error of the measurement value in terms of a percentage (e.g. accurate to within X%) or indicate the error as a range of values (e.g. the measurement value is accurate to $\pm$ Y), or indicate the error as a measure of the variance of the measurement value. In some embodiments, step 103 comprises estimating the error values via the variance of the measurement (determined according to classical statistical methods). In alternative embodiments, step 103 can comprise estimating the error values based on previous measurements (stored in a look up table). In other alternative embodiments, step 103 can comprise estimating the error values as a fixed value (e.g. X% of the respective measurement value). In another alternative embodiment, step 103 can comprise estimating the error values based on another measurement signal, for example if the sensor 20 detects body motion. For example, if there is a functional relationship with the measure of interest, e.g. y = f(x), the change of y can be estimated via dy = (df/dx)*dx, where df/dx is the derivative and dx the change of the variable x.

Next, in step 105, the set of blood pressure surrogate measurement values and the set of error values for the measurements are analysed using Bayesian inference to determine a trend in the blood pressure surrogate over time. Briefly, in step 105, the slope (gradient) needs to be determined taking into account the measurement error. This can be implemented by estimating the slope (gradient) *a* as representative of a trend given the obtained information from all data sources (i.e. the blood pressure surrogate measurement values obtained in step 101) including the estimated error (obtained or determined in step 103). Mathematically this can be expressed by:

$$p(a \mid data) = (p(data|a) * p(a))/p(data) \qquad (2)$$

with *p* representing the associated probabilities.

In an alternative embodiment, two hypotheses can be tested using the Bayesian technique by comparing the two hypotheses of a constant vs. a linear trend. This is expressed as:

$$O = \frac{p(C \mid data, I)}{p(M \mid data, I)} = \frac{p(data|C, I) * p(C, I)}{p(data|M, I) * p(M, I)} \qquad (3)$$

with *C* representing the hypothesis of a constant trend, *M* representing the hypothesis of a linear trend, and *I* representing any other information of relevance in the inference process such as the circadian rhythm of the subject, posture, stress etc. Dependent on the ratio of the calculated probabilities *O*, a decision can be made on the preference, e.g. by a threshold value, which hypothesis is preferred. Thus, for example, in this variant the output of step 105 can be an indication that the constant trend hypothesis is preferred, meaning that the blood pressure surrogate is following a constant trend (i.e. it is relatively static over time), or the output can be an indication that the linear trend hypothesis is preferred, meaning that the blood pressure surrogate is increasing or decreasing linearly.

As the blood pressure surrogate is a surrogate measurement for blood pressure, the blood pressure surrogate trend determined in step 105 provides a surrogate measurement of the trend in blood pressure.

It will be appreciated that the accuracy of the trend determined using Bayesian inference depends on the reliability of the blood pressure surrogate measurement values and the error values. Therefore, embodiments provide techniques for improving the reliability of the blood pressure surrogate measurement values by reducing the error values for those measurement values and/or by applying a correction to the blood pressure surrogate measurement values, and also for improving the estimation of the error values. These techniques take advantage of the fact that the blood pressure surrogate measurement values will be used for inferring a trend rather than determining an absolute blood pressure value. The errors in the measurements can be made up of systematic errors and random errors, which are both influential on the underlying trend. The provided techniques improve trend estimation for the blood pressure surrogate, and standardise measurement error.

Three embodiments are set out below, and they can be implemented individually or in any combination. The embodiments can all be implemented by the apparatus 12, and can be implemented between steps 103 and 105 of the method of Fig. 4. The first embodiment relates to the adjustment of the blood pressure surrogate measurement values and the corresponding error values according to the circadian rhythm of the subject. In this embodiment, the circadian rhythm of the subject is derived or estimated from a measurement of a physiological characteristic of the subject, such as heart rate and/or body temperature, and/or a measurement of the physical activity of the subject. The physiological characteristic may be measured by a device that is separate from the apparatus 12.

The second embodiment relates to standardising the error values via the blood pressure surrogate measurement process, for example by guiding the subject during the measurement process or by performing a standardised procedure or exercise before or during the measurement.

The third embodiment relates to correcting the blood pressure surrogate measurement values and/or the error values using measurements of another physiological characteristic, for example heart rate, to make different blood pressure surrogate measurements comparable. For example, the third embodiment can be used to compensate heart rate effects related to the pre-ejection period, PEP (where the PEP is the time interval from the beginning of the electrical stimulation of the ventricles to the opening of the aortic valve).

The three embodiments are described below primarily with reference to an implementation in a smart mirror (e.g. as shown in Fig. 3), but it will be appreciated that the embodiments can be implemented by apparatuses 12 having different form factors.

As noted above, the first embodiment relates to the adjustment of the blood pressure surrogate measurement values and the corresponding error values according to the circadian rhythm of the subject. In a particular implementation of the first embodiment, which is described below, the blood pressure surrogate is the PAT, but it will be appreciated that the first embodiment can be applied to other blood pressure surrogates that can vary according to the circadian rhythm of the subject.

Ideally, when determining a long term trend in blood pressure (e.g. a trend over weeks or months), measurements of the blood pressure surrogate are required every day, and the measurement of the blood pressure surrogate should be obtained at the same time during the day, since blood pressure surrogates and blood pressure are affected by the circadian rhythm of a subject. If measurements are obtained at different times of the day then obtaining a reliable trend measurement is a challenge. However, it may be that a subject is not able to take measurements according to this preferred scenario (e.g. they may work a varying shift pattern). Therefore, it is useful for a reliable estimation of an underlying trend in blood pressure using a blood pressure surrogate such as PAT as a surrogate to correct the acquired blood pressure surrogate (PAT) measurement values obtained at different times of the day to match them within the circadian rhythm before a trend analysis is performed.

Thus, a blood pressure surrogate (PAT) measurement has to be "offset" corrected for a systematic error in the blood pressure surrogate (PAT) measurement due to the effect of the circadian rhythm on blood pressure. In addition, the error value for the corrected blood pressure surrogate has to be corrected as well.

Firstly, it is necessary to estimate the circadian rhythm of the subject, and particularly estimate the point in the circadian rhythm/cycle that each blood pressure surrogate measurement value is obtained. An appropriate surrogate measure for estimating the circadian rhythm of the subject is the heart rate, body temperature and/or measure of the physical activity of the subject. To estimate the rhythm, the circadian rhythm surrogate measure should be acquired continuously throughout the day and night, and for example, the heart rate, body temperature and/or activity measure of the subject can be acquired continuously using a smart watch or other wearable that includes a PPG sensor, accelerometer or temperature sensor.

The circadian rhythm surrogate measure (i.e. the heart rate) is then used to correct blood pressure surrogate measurement values. For example, at the time that a PAT measurement is obtained, the surrogate measure of the circadian rhythm may indicate that the circadian rhythm is at a point where the PAT and blood pressure is higher than normal for the rest of the rhythm/cycle. Therefore, the blood pressure surrogate measurement value can be corrected by reducing the measurement value by an appropriate amount.

In some embodiments, the correction to be applied to the measurement value can be derived from or based on a linear function of the measure of the circadian rhythm (i.e. heart rate, body temperature and/or physical activity). That is, the correction can be derived from $(\alpha * S) + \beta$, where $S$ is the circadian rhythm surrogate measure and where $\alpha$ and $\beta$ are values to be determined based on other subjects (e.g. a population of subjects).

In alternative embodiments, the correction to be applied to the measurement value can be derived from or based on a mapping function between the point in the circadian rhythm and the required correction. A mapping function can be used if a linear function relating the correction to the circadian rhythm measurement is unknown or not applicable (i.e. if a linear function does not correctly describe the effect). The mapping function may be presented as a look-up table that provides an appropriate correction for a particular circadian rhythm surrogate measurement value.

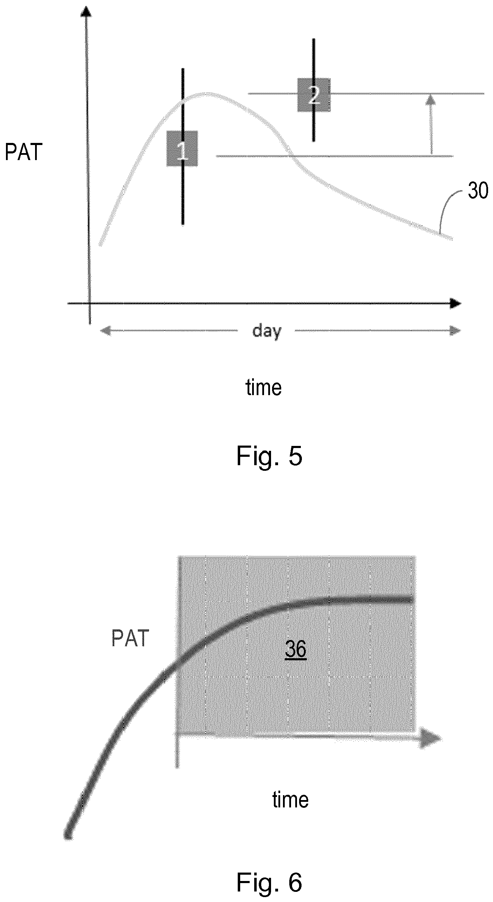

As another alternative, the method can include performing a calibration phase in which the surrogate measure of the circadian rhythm (i.e. heart rate, body temperature and/or physical activity level) is measured along with the blood pressure surrogate. The calibration phase can involve the measurement of the blood pressure surrogate, the actual blood pressure (for example using a cuff-based measurement device) and the circadian rhythm surrogate measure for a subject over a particular period of time (for example a week). These measurements can be compared and a correction required to blood pressure surrogate measurement values obtained at that point in the circadian rhythm determined. These predetermined corrections can then be used for subsequent blood pressure surrogate measurements. For example, the plot in Fig. 5 shows a trend line 30 that represents the blood pressure and shows a variation through a day due to the circadian rhythm, along with two measurements of PAT obtained at different times of the day, and therefore at different parts of the circadian rhythm. In this example, the second PAT measurement does not follow the expected circadian rhythm, and this can be taken into account in the error estimate of the second PAT measurement determined in step 103.

In yet another alternative, the correction required to the blood pressure surrogate measurement value based on a particular point of the circadian rhythm can be determined from data on the relationship between the blood pressure surrogate and the circadian rhythm for a population (e.g. for a general population, optionally with a weighting applied according to the specific data for the subject of interest, or for a population of subjects having a similar medical history or medical conditions to the subject of interest).

In addition to correcting the blood pressure surrogate measurement value for the timing of the measurement in the circadian rhythm, the error value for the measurement value can also be corrected. The correction for the error value can be determined, for example, via a look-up table that maps the surrogate measurement of the circadian rhythm to the required correction for the error value. In an embodiment where the circadian rhythm surrogate measure is heart rate, an increased heart rate value results in a higher error, since the subject might be stressed and the obtained surrogate is less reliable. The information in the look-up table can be determined from a functional relationship between error values and circadian rhythm surrogate measure obtained, for example, from population data (e.g. matched to the subject) with additional corrections for the specific condition or circumstances of the subject.

Turning now to the second embodiment, as noted above the second embodiment relates to standardising the blood pressure surrogate measurement process, for example by guiding the subject during the measurement process or by performing a standardised procedure or exercise before or during the measurement. This is because any movement or action by the subject (e.g. starting a PAT measurement or physical exertion) results in a short term arousal of the subject, which consequently results in a short term change in blood pressure and therefore a short term change in the blood pressure surrogate (e.g. PAT). Therefore measurements of the PAT to be used for determining a long term trend should be based on PAT measurement values obtained when the subject is at rest (and has been for some time) or more generally when the subject is in a defined health/activity state (e.g. when a specific exercise or activity is being performed). Otherwise, it is difficult to use individual blood pressure surrogate measurements to determine a long term trend.

The plot in Fig. 6 shows how the PAT of a subject varies over time after the subject starts to perform a physical activity, e.g. an exercise. It can be seen that the PAT increases over time and generally reaches a plateau (a steady-state). Thus, when measuring the PAT, ideally the measurement is made after the PAT reaches the plateau.

Therefore, in the second embodiment, such short term blood pressure surrogate/blood pressure effects need to be compensated for or mitigated by using appropriate measurement procedures which dampen and standardise the blood pressure surrogate acquisition process.

There are several ways in which the second embodiment can be implemented. In a first option, it is possible to evaluate measurements of the blood pressure surrogate obtained over a short time window (e.g. a few minutes) to determine if the blood pressure surrogate is in a steady state (i.e. constant). If so, a measurement value of the blood pressure surrogate can be obtained that can be used for the trend analysis in step 105. If not, then measurements of the blood pressure surrogate can continue to be taken until a steady state is reached. Thus, a real-time assessment of the obtained blood pressure surrogate measurement values (e.g. PAT) can be performed, and the time period in which the measurement values are being obtained is prolonged if the blood pressure surrogate is not in a steady state. A steady state can be identified by determining a rate of change of the blood pressure surrogate over time, and determining that the blood pressure surrogate is in a steady state if the magnitude of the rate of change of the blood pressure surrogate over a time period is less than a threshold value. In the example shown in Fig. 6, the PAT is increasing over time, and so a measurement value of the PAT to be used in the trend analysis may only be taken in the time period indicated by the shaded section 36. Prior to this time period, the PAT is not in a steady state, and so measurements taken at this time would not provide a reliable measure of PAT. As an alternative to waiting for a period of time for the blood pressure surrogate to reach a steady state (which may not be preferred as it can entail a longer period of time for which the subject has to be monitored), a set of blood pressure surrogate measurements can be obtained in a short period of time (e.g. 30s) and the steady state or 'plateau' can be estimated by extrapolation from those measurements. Those skilled in the art will be aware of various techniques that can be used to perform this extrapolation.

According to a second option, the subject can be instructed to perform a predefined exercise to standardise the blood pressure surrogate measurement process. In this way, the blood pressure surrogate measurement can be timed to occur some time after the start of the predefined exercise so that the measurement value is obtained when the subject is in a consistent state of arousal. In some embodiments, the apparatus 12 can provide the instructions to the subject to perform the predefined exercise. For example, instructions to perform the exercise can be presented on the mirror surface 24 (display screen) when the apparatus 12 is in the form of a smart mirror. Alternatively, instructions or guidance to perform the exercise can be presented on a(nother) display screen of the apparatus 12, or the instructions/guidance can be provided using an audible message (e.g. a pre-recorded spoken message). The instructions to perform the exercise may include instructing the subject to start performing the exercise (e.g. start relaxed breathing), or instructions that guide the subject through the exercise (e.g. breathe in slowly now, breathe out slowly now, etc.). The predefined exercise can include a breathing exercise, a hand gripping exercise, a posture-based exercise (e.g. relating to arm position), etc. In some embodiments, where the apparatus 12 includes a camera or other image/video capture device, the images from the camera or image/video capture device can be analysed by the processing unit 14 to identify the subject and their activities, movements and/or posture to verify if the subject is correctly performing the predefined exercise. In some embodiments, if the processing unit 14 determines that the subject is not performing the predefined exercise correctly, the processing unit 14 can determine feedback or further instructions/guidance for the subject to help or assist the subject to perform the exercise correctly.

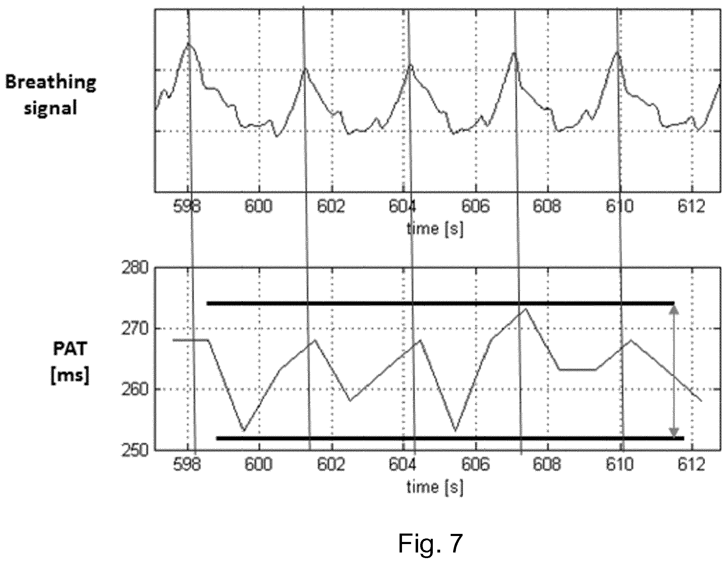

As an example of this second embodiment, it has been found that the PAT of a subject can be modulated with a variance of up to 15 to 20 milliseconds (ms) by breathing. This is illustrated in Fig. 7 which shows a signal indicating the breathing of a subject over time with an inhalation occurring approximately every 3 seconds (the top plot in Fig. 7). The bottom plot of Fig. 7 shows the PAT of the subject over the same time period as the breathing signal in the top plot of Fig. 7, and it can be seen that the PAT changes over time according to a pattern that is similar to the peaks and troughs in the breathing signal. Therefore, according to the second embodiment, a better (more reliable) PAT measurement value can be obtained if the subject is performing regular breathing and the PAT is measured only during a well-defined breathing phase (e.g. during expiration or at the end of an expiration). Alternatively the PAT can be measured across one or more full breathing cycles, and the obtained PAT measurements averaged to provide the PAT measurement value to use in the trend analysis in step 105. It will be appreciated that the breathing status of the subject can be measured using various different types of sensors, such as a camera, radar sensor or accelerometer.

As noted above, in the third embodiment the blood pressure surrogate measurement values and/or the error values are corrected using measurements of another physiological characteristic, for example heart rate, to make different blood pressure surrogate measurements comparable.

The PAT is the sum of the pre-ejection period (PEP) and the pulse transit time (PTT). The PTT is useful as a measure of a trend in blood pressure, but PEP is easily affected by the heart rate. Therefore the heart rate and heart rate changes have to be taken into account if PAT measurements are used to determine a trend.

Therefore, the 'PEP effect' can be compensated for by proper measurement of PTT changes and correcting for heart rate effects. According to the technical paper "Systolic Time Intervals in Heart Failure in Man" by Weissler et al., Circulation, Vol. XXXVII, No. 2 February 1968, PEP can be estimated from heart rate measurements as follows: *PEP:*

$$M \qquad PEP = -0.0004 * HR + 0.131 \qquad 0.013 \qquad (4)$$

$$F \qquad PEP = -0.0004 * HR + 0.133 \qquad 0.011 \qquad (5)$$

where *HR* is the heart rate, and M and F refer to the gender of the subject.

PAT can therefore be corrected for heart rate according to:

$$PTT = PAT - PEP = PAT - PEP_0 + \alpha * HR \qquad (6)$$

where $PEP_0$ is the value of PEP for a male (e.g. 0.131) or female (e.g. 0.133), and $\alpha$ is a gender specific factor indicated in the Weissler paper mentioned above. This procedure can help to reduce the effect of PEP due to heart rate and positively impacts the correlation between PTT and PAT and blood pressure.

An example illustrating the requirement to correct PAT for heart rate is shown below with reference to a set of 13 measurements. Table 1 shows the set of PAT measurements obtained over a period of 14 days, along with estimates of the PEP (in milliseconds, ms), heart rate (in beats per minute, bpm), and actual measurements of the blood pressure (both systolic and diastolic blood pressures).

Table 1

| Systolic | Diastolic | HR bpm | PEP ms | PAT Ms | PAT-PEP ms |
|----------|-----------|--------|--------|--------|------------|
| 120 | 89 | 87.5 | 98 | **259.87** | **161.87** |
| 114.5 | 89 | 105.5 | 90.8 | **270.50** | **179.70** |
| 115.5 | 90.5 | 127.5 | 82 | **241.61** | **159.61** |
| 116.5 | 86.5 | 106 | 90.6 | **272.30** | **181.70** |

(continued)

| Systolic | Diastolic | HR bpm | PEP ms | PAT Ms | PAT-PEP ms |
|---|---|---|---|---|---|
| 124.5 | 87 | 101.5 | 92.4 | **277.83** | **185.43** |
| 116 | 91 | 94.5 | 95.2 | **290.05** | **194.85** |
| 129.5 | 89.5 | 110.5 | 88.8 | **240.68** | **151.88** |
| 120.5 | 84.5 | 107 | 90.2 | **255.75** | **165.55** |
| 122.5 | 95 | 95 | 95 | **291.75** | **196.75** |
| 124.5 | 87.5 | 98.5 | 93.6 | **269.43** | **175.83** |
| 118 | 90.5 | 92 | 96.2 | **265.05** | **168.85** |
| 118 | 80.5 | 90.5 | 96.8 | **266.00** | **169.20** |
| 125 | 98.5 | 103 | 91.8 | **271.23** | **179.43** |

Thus each line of Table 1 shows a measurement of blood pressure (systolic and diastolic), heart rate, estimated PEP and measured PAT that were each obtained from the subject at approximately the same time on a particular day. It can be seen from the heart rate column that the heart rate of the subject at the time of the measurements varied from day to day, and overall the heart rate ranges from 87 bpm to 125 bpm. Due to these large heart rate differences, it can be seen that the PAT measurements show a dependency on heart rate. This dependency can be reduced by compensating the PEP according to the heart rate as discussed above. That is, the PAT and heart rate are measured, the PEP is estimated using the heart rate measurements according to equation (4) or (5) above, and the PTT is determined by subtracting the PEP estimate from the PAT measurement.

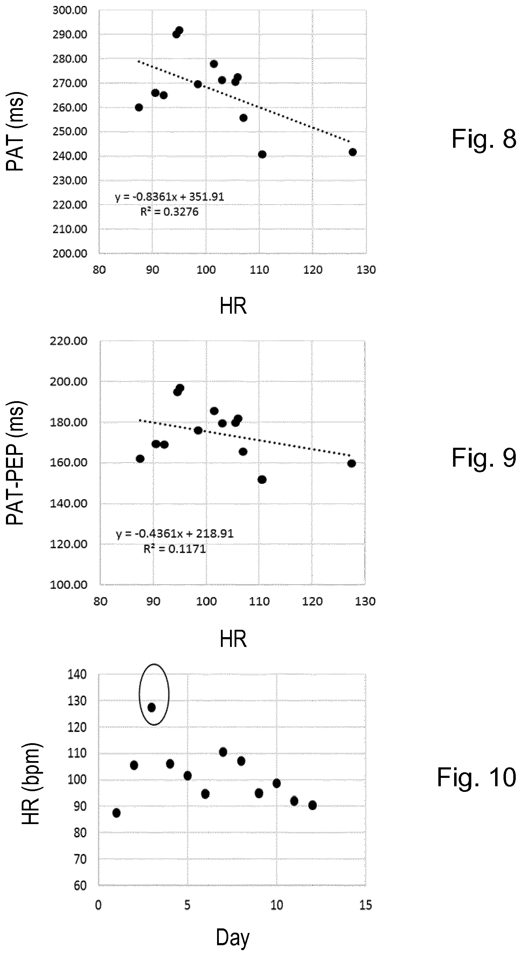

Figs. 8-13 show various different plots of the measurements in Table 1. In particular, Fig. 8 shows a plot of PAT against heart rate (HR), and this plot shows the dependency of PAT (due to the PEP) on heart rate, with the overall trend being that PAT decreases with increasing heart rate. Fig. 9 shows a plot of PAT-PEP against heart rate, and this plot shows that the correlation of PAT with heart rate after removal of PEP is significantly reduced. Thus, the correction via an estimated PEP effect with heart rate reduces the impact of heart rate on the blood pressure surrogate measurement, PAT.

This is more clearly seen in Figs. 10-13 where the 3rd measurement was acquired at a time where the heart rate was very high (~128 bpm), but this did not have an impact on the actual blood pressure, although it is visible in the PAT measurement. The correction of the PAT measurement according to an estimated PEP as discussed above will compensate for this variation in the heart rate for the measurements obtained over the 14-day period.

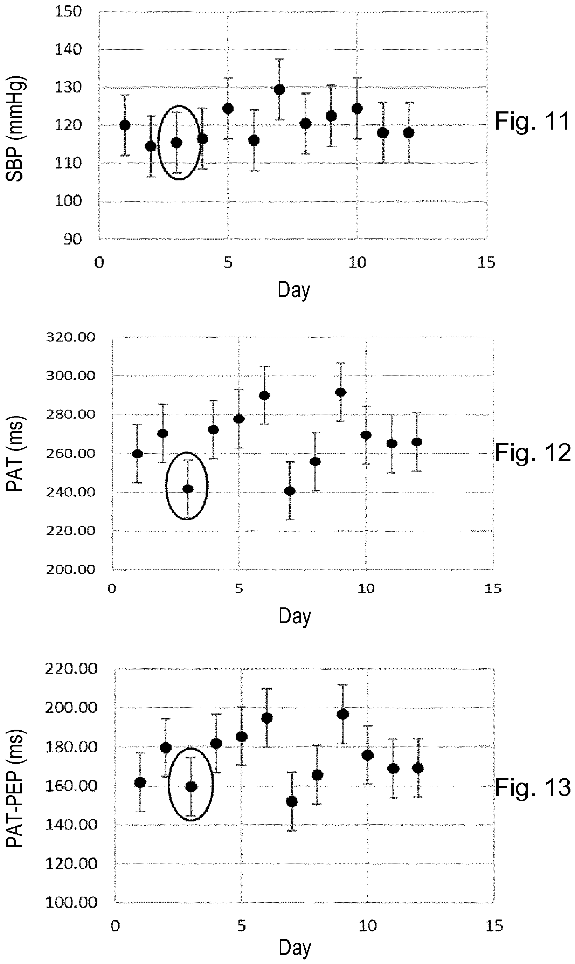

Thus, Fig. 10 is a plot of the heart rate measurements over time (one measurement per day), and it can be seen that the third measurement is an outlier in that it is much higher than the other 12 measurements. Fig. 11 is a plot of the systolic blood pressure (SBP) measurements in mmHg over time (again one measurement per day), and it can be seen that the systolic blood pressure measurement on the third day is similar to the systolic blood pressure measurements obtained on the preceding and subsequent days. The SBP measurements in Fig. 11 are assumed to have an error of 8 mmHg, as shown by the error bars. Fig. 12 is a plot of PAT over time (one measurement per day) and shows that the measurement on the third day is affected by the higher heart rate shown in Fig. 10. In Fig. 12 it is assumed that there is a constant error of 15 ms. Finally, Fig. 13 is a plot of PAT - PEP over time which shows the effect of correcting the PAT for the heart rate.

There is therefore provided improvements in the estimation of a trend from measurements of a blood pressure surrogate, particularly where the measurements of the blood pressure surrogate may have measurement errors.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A computer-implemented method of estimating a trend in a blood pressure surrogate, the method comprising:

   obtaining a set of blood pressure surrogate measurement values of a blood pressure surrogate for a subject;
   obtaining, for each blood pressure surrogate measurement value, an error value indicating a measurement error for the blood pressure surrogate measurement value; and
   analysing the set of blood pressure surrogate measurement values and the respective error values using Bayesian inference to determine a trend in the blood pressure surrogate over time.

2. A computer-implemented method as claimed in claim 1, wherein the step of analysing comprises:

   correcting each of the blood pressure surrogate measurement values in the set according to a point in a circadian rhythm of the subject at which each blood pressure surrogate measurement value was measured; and
   using Bayesian inference to determine the trend in the blood pressure surrogate over time by analysing the corrected blood pressure surrogate measurement values and the respective error values.

3. A computer-implemented method as claimed in claim 2, wherein the step of correcting comprises:

   obtaining measurements of a circadian rhythm surrogate of the subject;
   analysing the measurements of the circadian rhythm surrogate to identify a circadian rhythm of the subject;
   based on the identified circadian rhythm, determining respective points in the circadian rhythm at which each of the blood pressure surrogate measurement values in the set was measured;
   determining a respective correction for each of the blood pressure surrogate measurement values in the set based on the determined respective points in the circadian rhythm; and
   applying the determined respective corrections to the blood pressure surrogate measurement values in the set.

4. A computer-implemented method as claimed in any of claims 1-3, wherein the step of obtaining the set of blood pressure surrogate measurement values comprises, for each blood pressure surrogate measurement value:

   obtaining a plurality of blood pressure surrogate measurement values during a time window;
   evaluating the blood pressure surrogate measurement values obtained during the time window to determine if the blood pressure surrogate is in a steady state; and
   including a blood pressure surrogate measurement value obtained when the blood pressure surrogate is in a steady state in the set of blood pressure surrogate measurement values.

5. A computer-implemented method as claimed in any of claims 1-3, wherein the step of obtaining the set of blood pressure surrogate measurement values comprises, for each blood pressure surrogate measurement value:

   obtaining a plurality of blood pressure surrogate measurement values during a time window;
   evaluating the blood pressure surrogate measurement values obtained during the time window to determine if the blood pressure surrogate is in a steady state; and
   if the blood pressure surrogate is not in a steady state, extrapolating the blood pressure surrogate measurement values obtained during the time window to estimate the blood pressure surrogate measurement value in the steady state.

6. A computer-implemented method as claimed in any of claims 1-5, further comprising the step of:
   providing instructions to the subject to perform a procedure or exercise prior to, or during, a measurement of the blood pressure surrogate.

7. A computer-implemented method as claimed in any of claims 1-6, wherein the method is performed by a smart mirror that comprises one or more sensors for obtaining the set of blood pressure surrogate measurement values.

8. A computer program product comprising a computer readable medium having computer readable code embodied therein, the computer readable code being configured such that, on execution by a suitable computer or processor, the computer or processor is caused to perform the method of any of claims 1-7.

9. An apparatus for estimating a trend in a blood pressure surrogate, the apparatus comprising a processing unit configured to:

obtain a set of blood pressure surrogate measurement values of a blood pressure surrogate for a subject;
obtain, for each blood pressure surrogate measurement value, an error value indicating a measurement error for the blood pressure surrogate measurement value; and
analyse the set of blood pressure surrogate measurement values and the respective error values using Bayesian inference to determine a trend in the blood pressure surrogate over time.

10. An apparatus as claimed in claim 9, wherein the processing unit is configured to analyse the set of blood pressure surrogate measurement values and the respective error values using Bayesian inference to determine a trend in the blood pressure surrogate over time by:

correcting each of the blood pressure surrogate measurement values in the set according to a point in a circadian rhythm of the subject at which each blood pressure surrogate measurement value was measured; and
using Bayesian inference to determine the trend in the blood pressure surrogate over time by analysing the corrected blood pressure surrogate measurement values and the respective error values.

11. An apparatus as claimed in claim 10, wherein the processing unit is configured to correct each of the blood pressure surrogate measurement values in the set by:

obtaining measurements of a circadian rhythm surrogate of the subject;
analysing the measurements of the circadian rhythm surrogate to identify a circadian rhythm of the subject;
based on the identified circadian rhythm, determining respective points in the circadian rhythm at which each of the blood pressure surrogate measurement values in the set was measured;
determining a respective correction for each of the blood pressure surrogate measurement values in the set based on the determined respective points in the circadian rhythm; and
applying the determined respective corrections to the blood pressure surrogate measurement values in the set.

12. An apparatus as claimed in any of claims 9-11, wherein the processing unit is configured to obtain the set of blood pressure surrogate measurement values by, for each blood pressure surrogate measurement value:

obtaining a plurality of blood pressure surrogate measurement values during a time window;
evaluating the blood pressure surrogate measurement values obtained during the time window to determine if the blood pressure surrogate is in a steady state; and
including a blood pressure surrogate measurement value obtained when the blood pressure surrogate is in a steady state in the set of blood pressure surrogate measurement values.

13. An apparatus as claimed in any of claims 9-11, wherein the processing unit is configured to obtain the set of blood pressure surrogate measurement values by, for each blood pressure surrogate measurement value:

obtaining a plurality of blood pressure surrogate measurement values during a time window;
evaluating the blood pressure surrogate measurement values obtained during the time window to determine if the blood pressure surrogate is in a steady state; and
if the blood pressure surrogate is not in a steady state, extrapolating the blood pressure surrogate measurement values obtained during the time window to estimate the blood pressure surrogate measurement value in the steady state.

14. An apparatus as claimed in any of claims 9-13, wherein the processing unit is further configured to:
provide, via a user interface, instructions to the subject to perform a procedure or exercise prior to, or during, a measurement of the blood pressure surrogate.

15. An apparatus as claimed in any of claims 9-14, wherein the apparatus is a smart mirror that comprises one or more sensors for obtaining the set of blood pressure surrogate measurement values.

(a)

(b)

Fig. 1

20        12                      14

18                               16

Fig. 2

12

20a

24

22

20b

Fig. 3

Fig. 4

PAT

day

time

Fig. 5

PAT

36

time

Fig. 6

Fig. 7

EP 3 578 100 A1

Fig. 8

Fig. 9

Fig. 10

Fig. 11

Fig. 12

Fig. 13

Europäisches
Patentamt

European
Patent Office

Office européen
des brevets

# EUROPEAN SEARCH REPORT

Application Number

EP 18 17 5931

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2017/156084 A2 (UNIV ARIZONA STATE [US]; TAO NONGJIAN [US]; SHAO DANGDANG [US]) 14 September 2017 (2017-09-14) * paragraph [0066] - paragraph [0069] * * paragraph [0080] * * paragraph [0100] * * figures 1,2,14,15,17 * | 1-15 | INV. A61B5/021 A61B5/00 |
| A | US 2017/164904 A1 (KIRENKO IHOR OLEHOVYCH [NL]) 15 June 2017 (2017-06-15) * paragraph [0042] * * paragraph [0073] - paragraph [0074] * * paragraph [0098] * * figures 1,3 * | 1-15 | |
| A | US 9 462 980 B2 (INTEGRALIS LTD [IL]; INTEGRALIS LTD [IL]) 11 October 2016 (2016-10-11) * column 4, line 39 - line 40 * * column 17, line 50 - line 53 * * figure 6A * | 1-15 | |

TECHNICAL FIELDS
SEARCHED        (IPC)

A61B

The present search report has been drawn up for all claims

| Place of search | Date of completion of the search | Examiner |
|---|---|---|
| Berlin | 23 November 2018 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 3 578 100 A1

## ANNEX TO THE EUROPEAN SEARCH REPORT
## ON EUROPEAN PATENT APPLICATION NO.

EP 18 17 5931

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2018

| Patent document cited in search report | | Publication date | Patent family member(s) | | Publication date |
|---|---|---|---|---|---|
| WO 2017156084 | A2 | 14-09-2017 | NONE | | |
| US 2017164904 | A1 | 15-06-2017 | CA | 2931377 A1 | 04-06-2015 |
| | | | CN | 105792742 A | 20-07-2016 |
| | | | EP | 3073905 A1 | 05-10-2016 |
| | | | JP | 2016539697 A | 22-12-2016 |
| | | | US | 2017164904 A1 | 15-06-2017 |
| | | | WO | 2015078735 A1 | 04-06-2015 |
| US 9462980 | B2 | 11-10-2016 | AU | 2006271169 A1 | 25-01-2007 |
| | | | CA | 2614761 A1 | 25-01-2007 |
| | | | EP | 1904941 A2 | 02-04-2008 |
| | | | JP | 2009539416 A | 19-11-2009 |
| | | | US | 2009163776 A1 | 25-06-2009 |
| | | | US | 2015141771 A1 | 21-05-2015 |
| | | | WO | 2007010521 A2 | 25-01-2007 |
| | | | ZA | 200800489 B | 30-09-2009 |

EPO FORM P0459

For more details about this annex : see Official Journal of the European Patent Office, No. 12/82

24

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WEISSLER et al.** Systolic Time Intervals in Heart Failure in Man. *Circulation,* February 1968, vol. XXXVII (2